# EUROPEAN PATENT SPECIFICATION

(11) **EP 4 336 658 B1**
(45) Date of publication and mention of the grant of the patent: **12.11.2025**
(21) Application number: 23176622.1
(22) Date of filing: 01.06.2023
(51) Int. Cl.: H01R 4/50, H01R 4/58, H01R 12/63, H01R 12/70, A61B 5/00, A61B 5/01, A61B 5/0205

(54) **CONNECTOR**
VERBINDER
CONNECTEUR

(30) Priority: 12.09.2022 JP 2022144418
(43) Date of publication of application: 13.03.2024
(73) Proprietor: Japan Aviation Electronics Industry, Ltd., Tokyo 150-0043 (JP)
(72) Inventor: KOMOTO, Tetsuya, Tokyo, 150-0043 (JP); KINOSHITA, Takumi, Tokyo, 150-0043 (JP); NAKAMURA, Keisuke, Tokyo, 150-0043 (JP)
(74) Representative: Qip Patent & Recht Dr. Kuehn & Partner mbB

(56) References cited:
- DE-A1- 10 324 569
- JP-U- S5 851 581
- US-A1- 2007 224 878

## Description

### BACKGROUND OF THE INVENTION

The present invention relates to a connector, particularly to a connector for connecting a conductor portion of an electric wire to a flexible conductor of a sheet type conductive member.

In recent years, attention has been drawn to so-called smart clothes that can obtain user's biological data such as the heart rate and the body temperature only by being worn by the user. Such a smart cloth is equipped with an electrode disposed at a measurement position and formed of a flexible conductor, and when a wearable device serving as a measurement device is electrically connected to the electrode, it is possible to send biological data to the wearable device.

Connection between the electrode and the wearable device can be established by using, for example, a connector to be connected to the flexible conductor.

Meanwhile, in a case where the wearable device is disposed apart from a measurement position, it is necessary to constitute an electrical path from the electrode disposed at the measurement position to an attachment position of the connector, and when such an electrical path is formed of the flexible conductor, electric resistance becomes high, and cost increases.

To cope with it, in order to connect an electrode formed of a flexible conductor to a wearable device by an inexpensive electric wire with low electric resistance, the development of a small-sized connector for connecting an electric wire to a flexible conductor disposed on a garment is desired.

As a connector for connecting an electric wire to a flexible conductor, for instance, JP 2007-214087 A discloses a connector as shown in FIG. 50. The connector includes a first connector 2 connected to an end of a sheet type conductive member 1, and a second connector 4 attached to a tip end of an electric wire 3, and the second connector 4 is fitted with the first connector 2, whereby the electric wire 3 can be connected to a flexible conductor of the sheet type conductive member 1.

Since, however, the first connector 2 and the second connector 4 to be fitted with each other are required for connecting the electric wire 3 to the flexible conductor of the sheet type conductive member 1, the device is enlarged, while the electric connection is less reliable due to presence of a disconnectable connection site between the first connector 2 and the second connector 4.

DE 103 24 569 A1 discloses a device for connecting electrical conductors. US 2007/0224878 A1 discloses a method for contacting a flexible printed circuit with another flexible circuitry component and a related circuitries assembly.

### SUMMARY OF THE INVENTION

The present invention has been made to solve the conventional problem as above and aims at providing a connector that can connect a conductor portion of an electric wire to a flexible conductor of a sheet type conductive member with high reliability while its size can be reduced.

The connector according to the present invention is a connector as defined in claim 1 for connecting a conductor portion of an electric wire to a flexible conductor exposed on at least one surface of a sheet type conductive member.

### BRIEF DESCRIPTION OF THE DRAWINGS

FIG. 1 is a perspective view showing a connector according to Embodiment 1.
FIG. 2 is a plan view showing the connector according to Embodiment 1.
FIG. 3 is a front view showing the connector according to Embodiment 1.
FIG. 4 is a bottom view showing the connector according to Embodiment 1.
FIG. 5 is an exploded perspective view of the connector according to Embodiment 1.
FIG. 6 is a perspective view showing a first insulator used in the connector according to Embodiment 1.
FIG. 7 is a perspective view showing a second insulator used in the connector according to Embodiment 1 when viewed from an obliquely lower position.
FIG. 8 is a perspective view showing a contact force-securing member used in the connector according to Embodiment 1.
FIG. 9 is a cross-sectional view taken along line A-A in FIG. 2.
FIG. 10 is a cross-sectional view taken along line B-B in FIG. 2.
FIG. 11 is a cross-sectional view taken along line C-C in FIG. 3.
FIG. 12 is a perspective view showing a connector according to Embodiment 2.
FIG. 13 is a plan view showing the connector according to Embodiment 2.
FIG. 14 is a bottom view showing the connector according to Embodiment 2.
FIG. 15 is an exploded perspective view of the connector according to Embodiment 2.
FIG. 16 is a perspective view showing a first insulator used in the connector according to Embodiment 2.
FIG. 17 is a perspective view showing a second insulator used in the connector according to Embodiment 2 when viewed from an obliquely lower position.
FIG. 18 is a perspective view showing a relay member used in the connector according to Embodiment 2.
FIG. 19 is a cross-sectional view taken along line D-D in FIG. 13.
FIG. 20 is a cross-sectional view taken along line E-E in FIG. 13.
FIG. 21 is a perspective view showing a connector according to Embodiment 3.
FIG. 22 is a plan view showing the connector according to Embodiment 3.
FIG. 23 is a bottom view showing the connector according to Embodiment 3.
FIG. 24 is an exploded perspective view of the connector according to Embodiment 3.
FIG. 25 is a perspective view showing a second insulator used in the connector according to Embodiment 3 when viewed from an obliquely lower position.
FIG. 26 is a cross-sectional view taken along line F-F in FIG. 22.
FIG. 27 is a cross-sectional view taken along line G-G in FIG. 22.
FIG. 28 is a perspective view showing a connector according to Embodiment 4.
FIG. 29 is a plan view showing the connector according to Embodiment 4.
FIG. 30 is a bottom view showing the connector according to Embodiment 4.
FIG. 31 is an exploded perspective view of the connector according to Embodiment 4.
FIG. 32 is a perspective view showing a first insulator used in the connector according to Embodiment 4.
FIG. 33 is a perspective view showing a second insulator used in the connector according to Embodiment 4 when viewed from an obliquely lower position.
FIG. 34 is a cross-sectional view taken along line H-H in FIG. 29.
FIG. 35 is a cross-sectional view taken along line J-J in FIG. 29.
FIG. 36 is a perspective view showing a connector according to Embodiment 5.
FIG. 37 is a plan view showing the connector according to Embodiment 5.
FIG. 38 is an exploded perspective view of the connector according to Embodiment 5.
FIG. 39 is a perspective view showing a first insulator used in the connector according to Embodiment 5.
FIG. 40 is a perspective view showing a second insulator used in the connector according to Embodiment 5 when viewed from an obliquely lower position.
FIG. 41 is a cross-sectional view taken along line K-K in FIG. 37.
FIG. 42 is a cross-sectional view taken along line L-L in FIG. 37.
FIG. 43 is a perspective view showing a connector according to Embodiment 6.
FIG. 44 is a plan view showing the connector according to Embodiment 6.
FIG. 45 is an exploded perspective view of the connector according to Embodiment 6.
FIG. 46 is a perspective view showing a contact force-securing member used in the connector according to Embodiment 6.
FIG. 47 is a perspective view showing a second insulator used in the connector according to Embodiment 6 when viewed from an obliquely lower position.
FIG. 48 is a cross-sectional view taken along line M-M in FIG. 44.
FIG. 49 is a cross-sectional view taken along line N-N in FIG. 44.

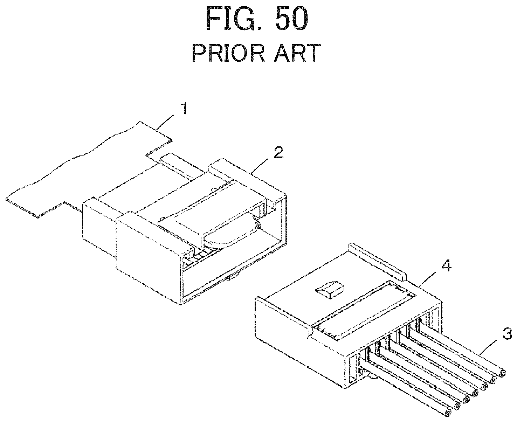

FIG. 50 is a perspective view showing a conventional connector.

### DETAILED DESCRIPTION OF THE INVENTION

Embodiments of the present invention are described below with reference to the accompanying drawings.

### Embodiment 1

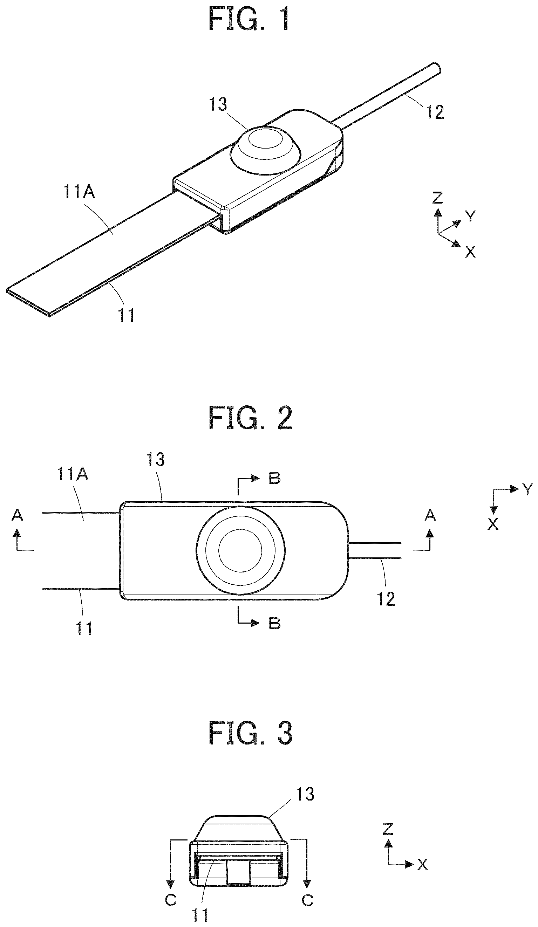

FIGS. 1 to 4 show a connector according to Embodiment 1. The connector is configured such that a covered electric wire 12 is connected to a sheet type conductive member 11, and the connector includes a housing 13 formed of an insulating resin material.

The sheet type conductive member 11 has a top surface and a bottom surface facing in opposite directions from each other and has a flexible conductor 11A exposed at least on the top surface. As the sheet type conductive member 11, conductive cloth woven using a conductive thread such as silver can be used, for example. When such conductive cloth is used, the flexible conductor 11A is exposed not only on the top surface but also on the bottom surface of the sheet type conductive member 11. In addition, one obtained by applying a conductive ink on a surface of cloth having no conductivity by printing or another method to form the flexible conductor 11A on the surface thereof can also be used as the sheet type conductive member 11. Further, a member obtained by forming the flexible conductor 11A formed of a conductive pattern on a surface of an insulating sheet body of a resin film or the like may be used as the sheet type conductive member 11.

The sheet type conductive member 11 has a band shape extending in a predetermined direction.

The covered electric wire 12 has such a structure that an outer periphery of a conductor portion described later is covered with an insulating cover portion. With the connector according to Embodiment 1, the conductor portion of the covered electric wire 12 is electrically connected to the flexible conductor 11A of the sheet type conductive member 11.

The covered electric wire 12 extends in the same direction as the direction in which the sheet type conductive member 11 of band shape extends.

For convenience, the predetermined direction in which the sheet type conductive member 11 extends toward the housing 13 is called "+Y direction," the width direction of the sheet type conductive member 11 of band shape "X direction," and the direction orthogonal to an XY plane "Z direction."

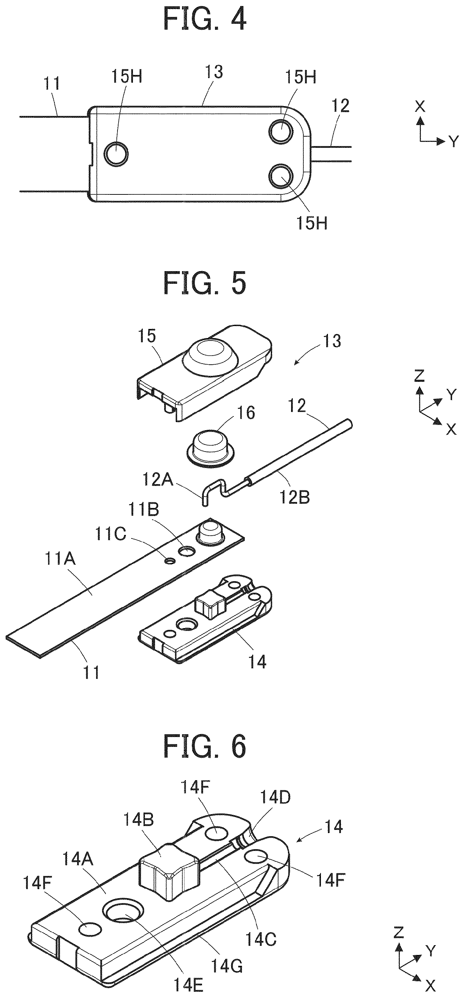

FIG. 5 shows an exploded perspective view of the connector. The connector includes a first insulator 14 and a second insulator 15, and these first and second insulators 14 and 15 constitute the housing 13.

The sheet type conductive member 11 is disposed on the +Z direction side of the first insulator 14, and a conductor portion 12A exposed from an insulating cover portion 12B of the covered electric wire 12 is disposed on the +Z direction side of the sheet type conductive member 11. The conductor portion 12A of the covered electric wire 12 may be either of a so-called single line that is formed of one conductor line and a so-called strand that is formed by twisting a plurality of conductors.

In addition, the connector includes a contact force-securing member 16. The contact force-securing member 16 is disposed on the +Z direction side of the conductor portion 12A of the covered electric wire 12, and the second insulator 15 is disposed on the +Z direction side of the contact force-securing member 16.

As shown in FIG. 6, the first insulator 14 includes a flat plate portion 14A of substantially rectangular shape extending along an XY plane, and a protrusion portion 14B of substantially prismatic shape protruding in the +Z direction is formed on the +Z directional surface (first retaining surface) of the flat plate portion 14A.

On the +Z directional surface of the flat plate portion 14A, an insertion groove 14C is formed to extend in the +Y direction from the protrusion portion 14B, and at the +Y directional end of the insertion groove 14C, an insertion groove 14D is formed with a larger groove width than that of the insertion groove 14C.

Further, at a portion of the flat plate portion 14A on the -Y direction side of the protrusion portion 14B, a projection housing portion 14E of recess shape is formed to be recessed in the -Z direction.

The flat plate portion 14A includes three through-holes 14F separately formed on opposite sides of the insertion groove 14C in the X direction and on the -Y direction side of the projection housing portion 14E, the through-holes 14F penetrating the flat plate portion 14A in the Z direction.

In addition, at the X-directional opposite lateral surfaces of the flat plate portion 14A, step portions 14G are separately formed to extend in the Y direction.

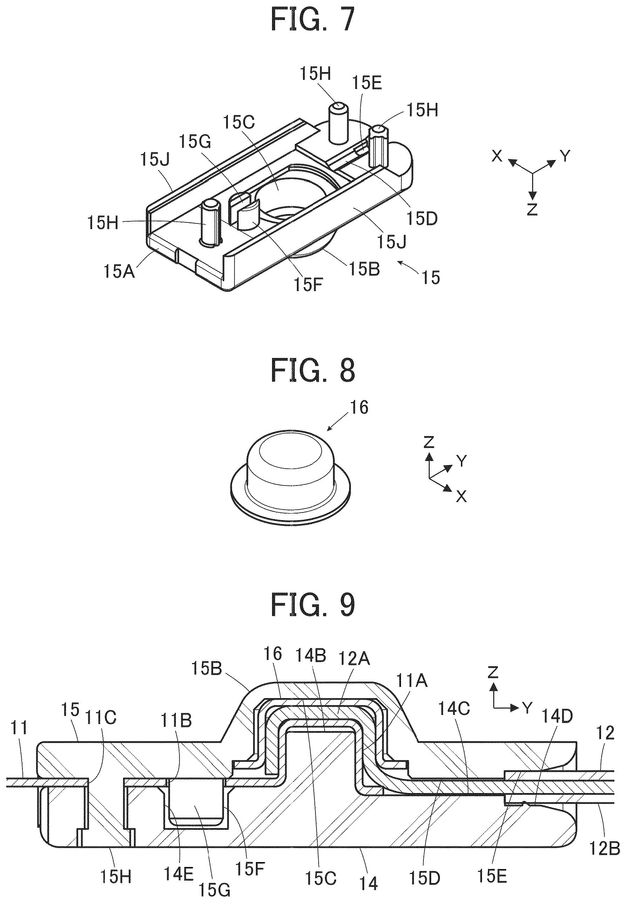

As shown in FIG. 7, the second insulator 15 includes a flat plate portion 15A of substantially rectangular shape extending along an XY plane, and a dome-shaped portion 15B protruding in the +Z direction from the flat plate portion 15A. A recess portion 15C is formed to extend from the -Z directional surface (second retaining surface) of the flat plate portion 15A to an inside of the dome-shaped portion 15B and open toward the -Z direction.

On the -Z directional surface of the flat plate portion 15A, an insertion groove 15D is formed to extend in the +Y direction from the recess portion 15C, and at the +Y directional end of the insertion groove 15D, an insertion groove 15E is formed with a larger groove width than that of the insertion groove 15D.

Further, at a portion of the flat plate portion 15A on the -Y direction side of the recess portion 15C, a projection 15F is formed to protrude in the -Z direction, and a slit 15G is formed in the projection 15F to extend in the Y direction so as to divide the projection 15F in two in the X direction. The slit 15G is to serve as a tip retaining portion for retaining a tip of the conductor portion 12A of the covered electric wire 12 and has a slit width that is slightly smaller than the diameter of the conductor portion 12A.

The flat plate portion 15A includes three bosses 15H separately formed on opposite sides of the insertion groove 15D in the X direction and on the -Y direction side of the projection 15F, the bosses 15H protruding in the -Z direction.

In addition, at the X-directional opposite lateral portions of the flat plate portion 15A, lateral plates 15J are separately formed to protrude in the -Z direction and extend in the Y direction.

When the first insulator 14 and the second insulator 15 are connected to each other, the insertion groove 14C of the first insulator 14 and the insertion groove 15D of the second insulator 15 work together to retain the conductor portion 12A of the covered electric wire 12, and the insertion groove 14D of the first insulator 14 and the insertion groove 15E of the second insulator 15 work together to retain the insulating cover portion 12B of the covered electric wire 12 so as to constitute an insulating cover retaining portion.

As shown in FIG. 5, the sheet type conductive member 11 is provided with through-holes 11B and 11C respectively corresponding to the projection 15F of the second insulator 15 and the boss 15H on the -Y direction side of the second insulator 15.

The contact force-securing member 16 is formed of a metal material and has a cylindrical shape as shown in FIG. 8. The contact force-securing member 16 is, as shown in FIG. 9, disposed at an outside of the conductor portion 12A of the covered electric wire 12 and the flexible conductor 11A of the sheet type conductive member 11, the conductor portion 12A and the flexible conductor 11A contacting each other, in the recess portion 15C of the second insulator 15, and secures the contact force between the conductor portion 12A and the flexible conductor 11A.

To assemble the connector as above, the contact force-securing member 16 is first inserted in the recess portion 15C of the second insulator 15 from the -Z direction, and the tip of the conductor portion 12A exposed as a result of removal of a portion of the insulating cover portion 12B at the -Y directional end of the covered electric wire 12 is press-fitted in the slit 15G formed as the tip retaining portion in the projection 15F of the second insulator 15, thereby being temporarily retained in the slit 15G.

In this state, the first insulator 14 is pressed toward the second insulator 15 relatively in the +Z direction with the sheet type conductive member 11 being held therebetween. Accordingly, the sheet type conductive member 11 and the conductor portion 12A of the covered electric wire 12 are held between the +Z directional surface (first retaining surface) of the first insulator 14 and the -Z directional surface (second retaining surface) of the second insulator 15.

At this time, owing to the protrusion portion 14B having a substantially prismatic shape of the first insulator 14, the sheet type conductive member 11 and the conductor portion 12A of the covered electric wire 12 are pressed and inserted in an inside of the contact force-securing member 16 disposed in the recess portion 15C of the second insulator 15.

In addition, when the first insulator 14 is pressed against the second insulator 15, the three bosses 15H of the second insulator 15 penetrate the three through-holes 14F of the first insulator 14. In this process, the boss 15H positioned on the -Y direction side among the three bosses 15H penetrates the corresponding through-hole 14F of the first insulator 14 via the through-hole 11C of the sheet type conductive member 11 shown in FIG. 5.

As shown in FIG. 4, tips of the three bosses 15H projecting on the -Z direction side of the first insulator 14 are then thermally deformed, whereby the first insulator 14 and the second insulator 15 are fixed to each other. Thus, the assembling operation of the connector is completed.

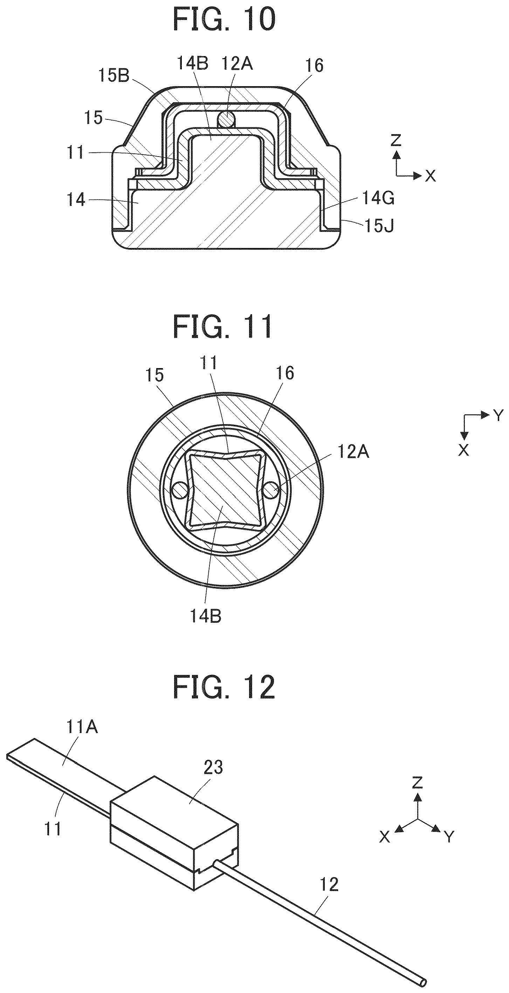

The protrusion portion 14B of the first insulator 14 is inserted in the inside of the contact force-securing member 16 while pressing the sheet type conductive member 11 and the conductor portion 12A of the covered electric wire 12 therein, whereby, as shown in FIGS. 9 and 10, the sheet type conductive member 11 deforms to conform to a surface of the protrusion portion 14B, and the conductor member 12A of the covered electric wire 12 is held between a surface of the sheet type conductive member 11 and an inner surface of the contact force-securing member 16 to be bent to conform to the surface of the deformed sheet type conductive member 11.

Since the protrusion portion 14B has a substantially prismatic shape as shown in FIG. 11, the sheet type conductive member 11 deformed to conform to the surface of the protrusion portion 14B and the conductor portion 12A of the covered electric wire 12 are held between the +Y and -Y directional lateral surfaces of the protrusion portion 14B and the inner surface of the contact force-securing member 16 of cylindrical shape, and the conductor portion 12A of the covered electric wire 12 contacts the flexible conductor 11A exposed on the top surface of the sheet type conductive member 11 at a predetermined contact force and is electrically connected to the flexible conductor 11A.

In the meantime, the conductor portion 12A of the covered electric wire 12 bends to conform to the surface of the deformed sheet type conductive member 11, whereby the tip of the conductor portion 12A temporarily retained in the slit 15G of the projection 15F of the second insulator 15 is pulled out from the slit 15G as shown in FIG. 9.

When the first insulator 14 and the second insulator 15 are connected to each other, as shown in FIG. 9, the projection 15F of the second insulator 15 penetrates the through-hole 11B of the sheet type conductive member 11 and is housed in the projection housing portion 14E of the first insulator 14, and the conductor portion 12A of the covered electric wire 12 is retained by the insertion groove 14C of the first insulator 14 and the insertion groove 15D of the second insulator 15, while the insulating cover portion 12B of the covered electric wire 12 is retained by the insertion groove 14D of the first insulator 14 and the insertion groove 15E of the second insulator 15.

In addition, as shown in FIG. 10, the pair of lateral plates 15J of the second insulator 15 are fitted in the pair of step portions 14G of the first insulator 14.

In the connector according to Embodiment 1, the protrusion portion 14B of the first insulator 14 is inserted in the inside of the contact force-securing member 16 that is inserted in the recess portion 15C of the second insulator 15 while pressing the sheet type conductive member 11 and the conductor portion 12A of the covered electric wire 12 therein, whereby the flexible conductor 11A exposed at the top surface of the sheet type conductive member 11 is electrically connected to the conductor portion 12A of the covered electric wire 12. Hence, the connector can reduce the size thereof while improving reliability of the electric connection between the flexible conductor 11A and the conductor portion 12A.

When the connector of Embodiment 1 is applied to a smart cloth, and an electrode (not shown) is connected to the flexible conductor 11A of the sheet type conductive member 11, the electrode disposed at a measurement position and a wearable device can be connected to each other by means of the inexpensive covered electric wire 12 with low electric resistance.

By using a water-resistant adhesive to seal between the first insulator 14 and the second insulator 15, it is possible to configure a waterproof connector that prevents entry of water into a site of electric connection between the flexible conductor 11A of the sheet type conductive member 11 and the conductor portion 12A of the covered electric wire 12.

While in Embodiment 1 described above, the slit 15G formed in the projection 15F of the second insulator 15 constitutes the tip retaining portion for retaining the tip of the conductor portion 12A of the covered electric wire 12, the tip retaining portion may be formed in the first insulator 14.

In addition, while in Embodiment 1 described above, the three bosses 15H of the second insulator 15 penetrate the three through-holes 14F of the first insulator 14, it is possible to configure the connector in which, conversely, a plurality of bosses 15H formed in the first insulator 14 penetrate a plurality of through-holes formed in the second insulator 15.

### Embodiment 2

FIGS. 12 to 14 show a connector according to Embodiment 2. As with the connector according to Embodiment 1, this connector is configured such that the covered electric wire 12 is connected to the sheet type conductive member 11, and the connector includes a housing 23 formed of an insulating resin material.

The sheet type conductive member 11 and the covered electric wire 12 are the same as those used in Embodiment 1.

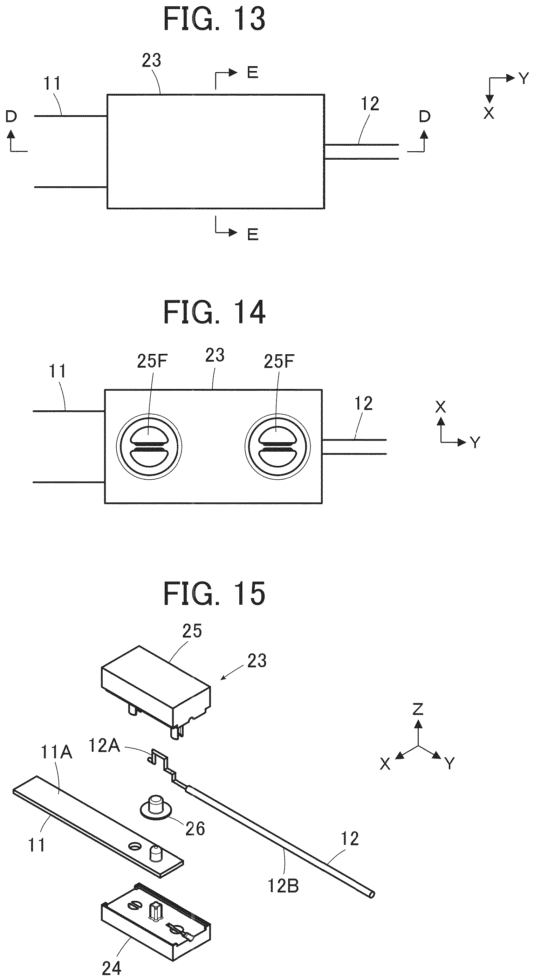

FIG. 15 shows an exploded perspective view of the connector according to Embodiment 2. The connector includes a first insulator 24 and a second insulator 25, and these first and second insulators 24 and 25 constitute the housing 23.

The sheet type conductive member 11 is disposed on the +Z direction side of the first insulator 24, a relay member 26 is disposed on the +Z direction side of the sheet type conductive member 11, and the conductor portion 12A exposed from the insulating cover portion 12B of the covered electric wire 12 is disposed on the +Z direction side of the relay member 26.

Further, the second insulator 25 is disposed on the +Z direction side of the conductor portion 12A of the covered electric wire 12.

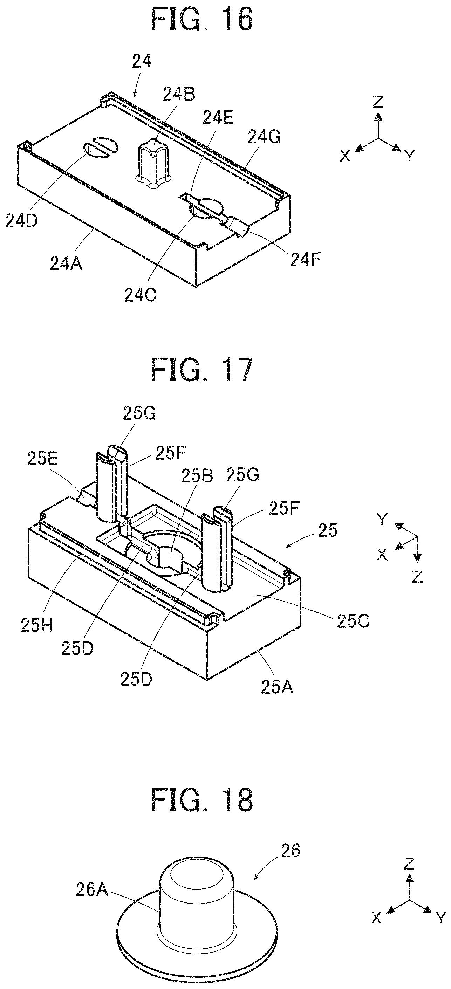

As shown in FIG. 16, the first insulator 24 includes a flat plate portion 24A of substantially rectangular shape extending along an XY plane, and a protrusion portion 24B of substantially prismatic shape protruding in the +Z direction is formed on the +Z directional surface (first retaining surface) of the flat plate portion 24A.

The flat plate portion 24A is provided with a pair of through-holes 24C spaced apart from each other in the X direction on the +Y direction side of the protrusion portion 24B and a pair of through-holes 24D also spaced apart from each other in the X direction on the -Y direction side of the protrusion portion 24B.

On a surface of a portion, on the +Y direction side of the protrusion portion 24B, of the flat plate portion 24A, an insertion groove 24E is formed to extend in the +Y direction between the pair of through-holes 24C, and at the +Y directional end of the insertion groove 24E, an insertion groove 24F is formed with a larger groove width than that of the insertion groove 24E.

In addition, at the X-directional opposite lateral ends of the flat plate portion 24A, jutting portions 24G are separately formed to protrude in the +Z direction and extend in the Y direction.

As shown in FIG. 17, the second insulator 25 includes a flat plate portion 25A of substantially rectangular shape extending along an XY plane, and a recess portion 25B is formed at the middle in the Y direction of the flat plate portion 25A and recessed in the +Z direction from the -Z directional surface (second retaining surface) of the flat plate portion 25A.

On the -Z directional surface of the flat plate portion 25A, a sheet type conductive member housing portion 25C of recess shape is formed for housing the +Y directional end of the sheet type conductive member 11. The sheet type conductive member housing portion 25C extends from the +Y direction side of the recess portion 25B to the -Y directional end of the flat plate portion 25A.

On the surface of the flat plate portion 25A, an insertion groove 25D is formed to extend in the +Y direction and the -Y direction from the recess portion 25B, and at the +Y directional end of the insertion groove 25D extending in the +Y direction from the recess portion 25B, an insertion groove 25E is formed with a larger groove width than that of the insertion groove 25D.

The flat plate portion 25A includes two bosses 25F separately formed on the +Y direction side and the -Y direction side of the recess portion 25B, the bosses 25F protruding in the -Z direction. In each of the bosses 25F, a slit 25G is formed to extend in the Y direction so as to divide the relevant boss 25F in two in the X direction. The slits 25G are connected to the insertion groove 25D.

In addition, at the X-directional opposite lateral ends of the flat plate portion 25A, step portions 25H are separately formed to extend in the Y direction.

The relay member 26 is formed of a conductive metal material and includes a tubular portion 26A of cylindrical shape as shown in FIG. 18. The relay member 26 is disposed between the conductor portion 12A of the covered electric wire 12 and the flexible conductor 11A of the sheet type conductive member 11 in the recess portion 25B of the second insulator 25, and is used to electrically connect the conductor portion 12A to the flexible conductor 11A.

To assemble the connector according to Embodiment 2, the conductor portion 12A of the covered electric wire 12 is first inserted in the slits 25G of the two bosses 25F of the second insulator 25 from the -Z direction, and in this state, the first insulator 24 is pressed toward the second insulator 25 relatively in the +Z direction with the relay member 26 and the sheet type conductive member 11 being held therebetween. Accordingly, the sheet type conductive member 11 and the conductor portion 12A of the covered electric wire 12 are held between the +Z directional surface (first retaining surface) of the first insulator 24 and the -Z directional surface (second retaining surface) of the second insulator 25.

At this time, owing to the protrusion portion 24B having a substantially prismatic shape of the first insulator 24, the sheet type conductive member 11, the relay member 26, and the conductor portion 12A are pressed in the recess portion 25B of the second insulator 25.

In addition, when the first insulator 24 is pressed against the second insulator 25, the two bosses 25F of the second insulator 25 penetrate the through-holes 24C and 24D of the first insulator 24. In this process, among the two bosses 25F, the boss 25F positioned on the +Y direction side penetrates the through-hole 24C that is divided in two by the slit 25G, and the boss 25F positioned on the -Y direction side penetrates the through-hole 24D that is divided in two by the slit 25G.

As shown in FIG. 14, tips of the two bosses 25F protruding on the -Z direction side of the first insulator 24 are then thermally deformed, whereby the first insulator 24 and the second insulator 25 are fixed to each other. Thus, the assembling operation of the connector is completed.

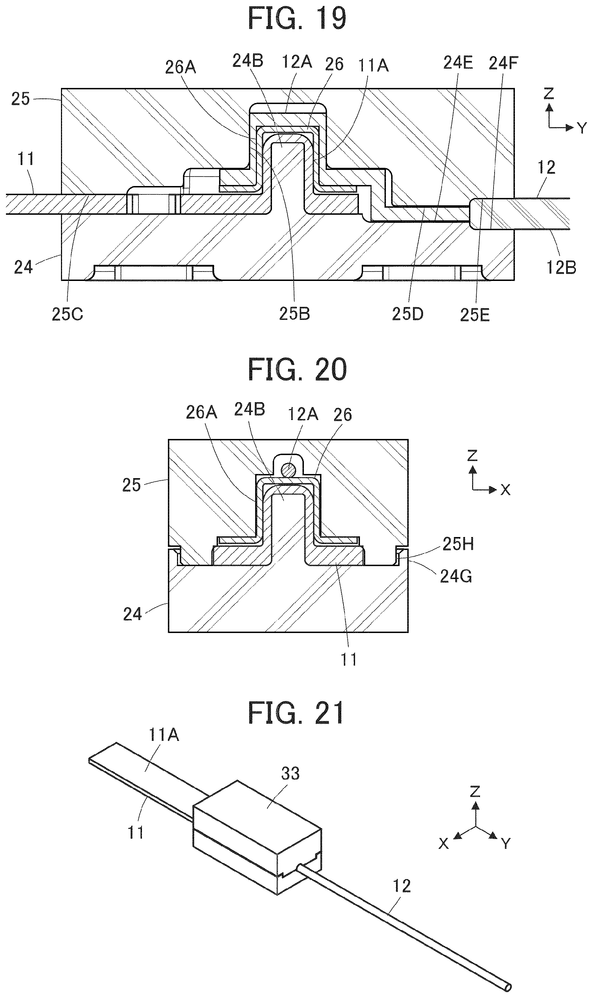

The protrusion portion 24B of the first insulator 24 presses the sheet type conductive member 11, the relay member 26, and the conductor portion 12A into the recess portion 25B of the second insulator 25, whereby, as shown in FIGS. 19 and 20, the sheet type conductive member 11 deforms to conform to a surface of the protrusion portion 24B and is held between an outer surface of the protrusion portion 24B and an inner surface of the tubular portion 26A of the relay member 26. In addition, the conductor portion 12A of the covered electric wire 12 is held between an outer surface of the tubular portion 26A of the relay member 26 and an inner surface of the recess portion 25B of the second insulator 25.

Thus, the conductor portion 12A of the covered electric wire 12 is electrically connected to the flexible conductor 11A exposed on the top surface of the sheet type conductive member 11 via the relay member 26.

The sheet type conductive member 11 is housed in the sheet type conductive member housing portion 25C of the second insulator 25, the conductor portion 12A of the covered electric wire 12 is retained in the insertion groove 24E of the first insulator 24 and the insertion groove 25D of the second insulator 25, and the insulating cover portion 12B of the covered electric wire 12 is retained in the insertion groove 24F of the first insulator 24 and the insertion groove 25E of the second insulator 25.

In addition, as shown in FIG. 20, the pair of jutting portions 24G of the first insulator 24 are fitted in the pair of step portions 25H of the second insulator 25.

As with Embodiment 1, the connector according to Embodiment 2 can also reduce the size thereof while improving reliability of the electric connection between the flexible conductor 11A and the conductor portion 12A.

### Embodiment 3

While the conductor portion 12A of the covered electric wire 12 is electrically connected to the flexible conductor 11A of the sheet type conductive member 11 via the relay member 26 in Embodiment 2 described above, the conductor portion 12A of the covered electric wire 12 can be brought into direct contact with and electrically connected to the flexible conductor 11A of the sheet type conductive member 11 without having the relay member 26 therebetween, similarly to Embodiment 1.

FIGS. 21 to 23 show a connector according to Embodiment 3. As with the connectors according to Embodiments 1 and 2, this connector is configured such that the covered electric wire 12 is connected to the sheet type conductive member 11, and the connector includes a housing 33 formed of an insulating resin material.

The sheet type conductive member 11 and the covered electric wire 12 are the same as those used in Embodiments 1 and 2.

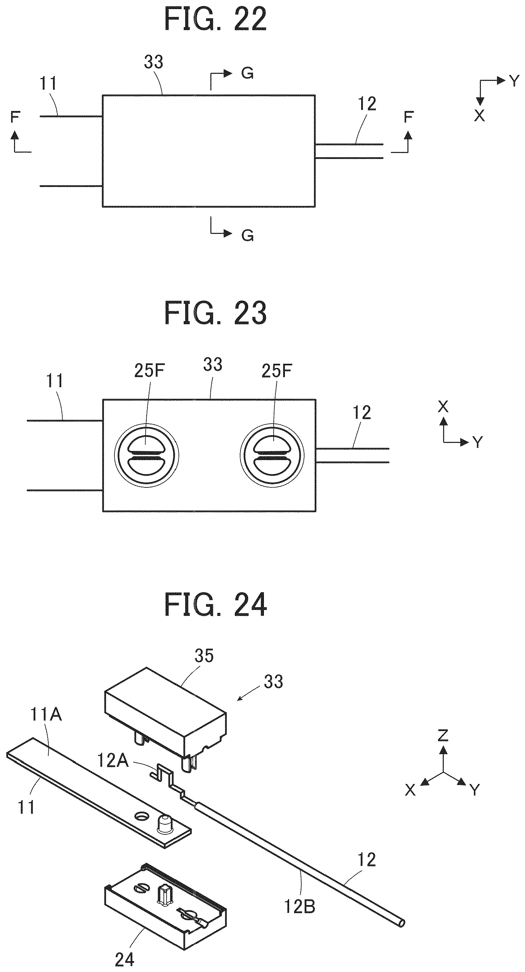

FIG. 24 shows an exploded perspective view of the connector according to Embodiment 3. The connector includes the first insulator 24 used in Embodiment 2 and a second insulator 35, and these first and second insulators 24 and 35 constitute the housing 33.

The sheet type conductive member 11 is disposed on the +Z direction side of the first insulator 24, the conductor portion 12A of the covered electric wire 12 is disposed on the +Z direction side of the sheet type conductive member 11, and the second insulator 35 is disposed on the +Z direction side of the conductor portion 12A.

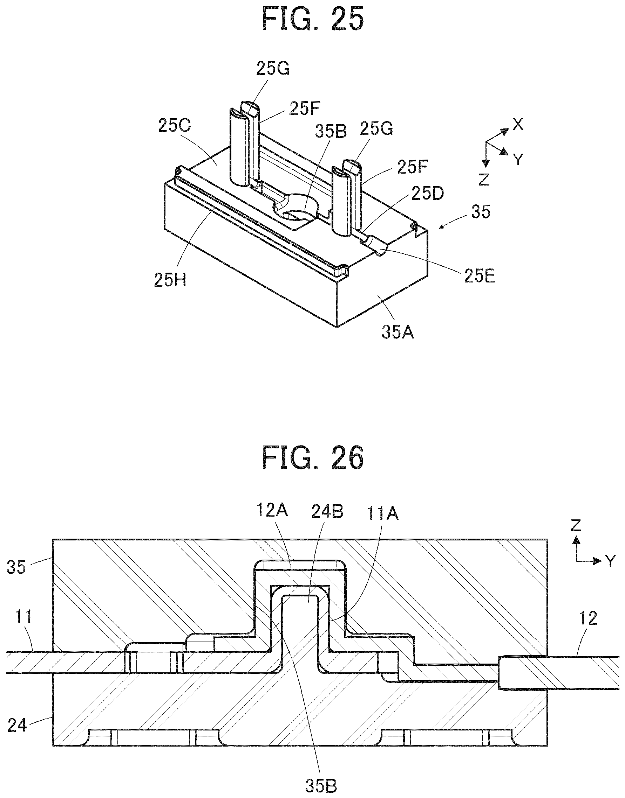

As shown in FIG. 25, as with the second insulator 25 in Embodiment 2, the second insulator 35 includes a flat plate portion 35A in which a recess portion 35B is formed.

Similarly to the second insulator 25 in Embodiment 2, the flat plate portion 35A includes the sheet type conductive member housing portion 25C of recess shape, the insertion grooves 25D and 25E, the two bosses 25F each having the slit 25G, and the pair of step portions 25H.

To assemble the connector according to Embodiment 3, the conductor portion 12A of the covered electric wire 12 is first inserted in the slits 25G of the two bosses 25F of the second insulator 35 from the -Z direction, and in this state, the first insulator 24 is pressed toward the second insulator 35 relatively in the +Z direction with the sheet type conductive member 11 being held therebetween. Accordingly, the sheet type conductive member 11 and the conductor portion 12A of the covered electric wire 12 are held between the +Z directional surface (first retaining surface) of the first insulator 24 and the -Z directional surface (second retaining surface) of the second insulator 35.

At this time, owing to the protrusion portion 24B having a substantially prismatic shape of the first insulator 24, the sheet type conductive member 11 and the conductor portion 12A are pressed in the recess portion 35B of the second insulator 35.

When the first insulator 24 is pressed against the second insulator 35, the two bosses 25F of the second insulator 35 penetrate the through-holes 24C and 24D of the first insulator 24, and as shown in FIG. 23, tips of the two bosses 25F protruding on the -Z direction side of the first insulator 24 are thermally deformed, whereby the first insulator 24 and the second insulator 35 are fixed to each other. Thus, the assembling operation of the connector is completed.

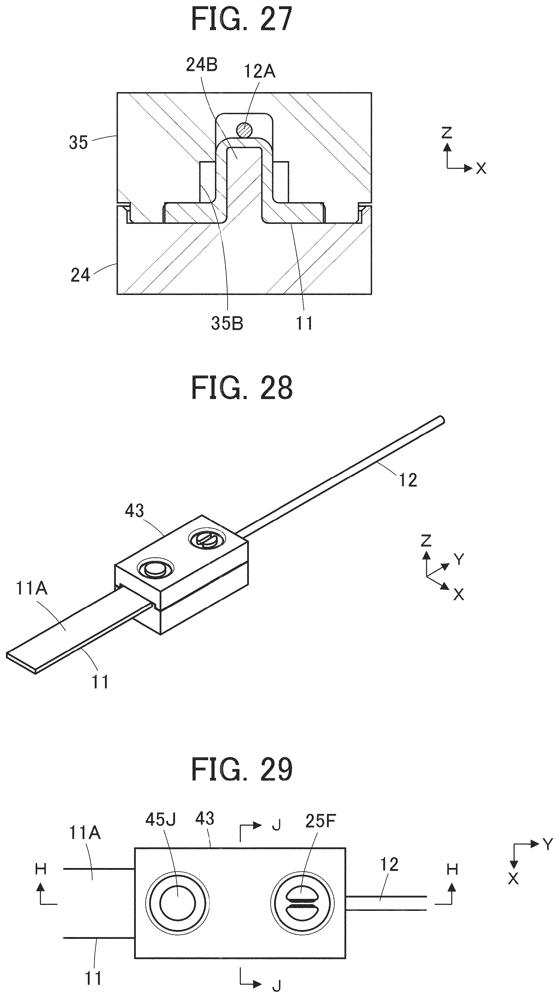

The protrusion portion 24B of the first insulator 24 presses the sheet type conductive member 11 and the conductor portion 12A into the recess portion 35B of the second insulator 35, whereby, as shown in FIGS. 26 and 27, the sheet type conductive member 11 and the conductor portion 12A deform to conform to a surface of the protrusion portion 24B and are held between an outer surface of the protrusion portion 24B and an inner surface of the recess portion 35B of the second insulator 35.

Thus, the conductor portion 12A of the covered electric wire 12 is brought into direct contact with and electrically connected to the flexible conductor 11A exposed on the top surface of the sheet type conductive member 11.

As with Embodiments 1 and 2, the connector according to Embodiment 3 can also reduce the size thereof while improving reliability of the electric connection between the flexible conductor 11A and the conductor portion 12A.

### Embodiment 4

While the sheet type conductive member 11 is inserted to an inside of the tubular portion 26A of the relay member 26, and the conductor portion 12A of the covered electric wire 12 is disposed on an outside of the relay member 26 in Embodiment 2 described above, even when, conversely, the conductor portion 12A of the covered electric wire 12 is inserted to an inside of the tubular portion 26A of the relay member 26, and the sheet type conductive member 11 is disposed on an outside of the relay member 26, the conductor portion 12A of the covered electric wire 12 can be electrically connected to the flexible conductor 11A of the sheet type conductive member 11 via the relay member 26.

FIGS. 28 to 30 show a connector according to Embodiment 4. As with the connectors according to Embodiments 1 to 3, this connector is configured such that the covered electric wire 12 is connected to the sheet type conductive member 11, and the connector includes a housing 43 formed of an insulating resin material.

The sheet type conductive member 11 and the covered electric wire 12 are the same as those used in Embodiments 1 to 3.

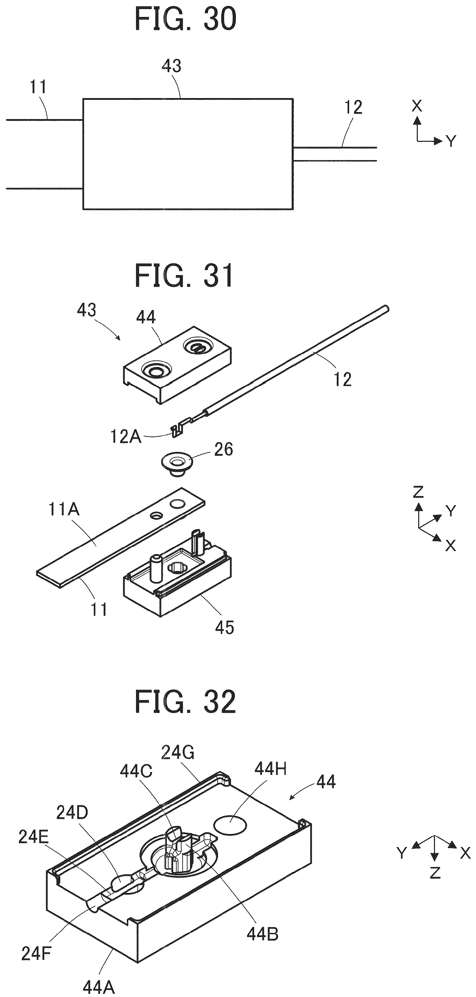

FIG. 31 shows an exploded perspective view of the connector according to Embodiment 4. The connector includes a first insulator 44 and a second insulator 45, and these first and second insulators 44 and 45 constitute the housing 43.

The conductor portion 12A exposed from the insulating cover portion 12B of the covered electric wire 12 is disposed on the -Z direction side of the first insulator 44, the relay member 26 used in Embodiment 2 is disposed on the -Z direction side of the conductor portion 12A, and the sheet type conductive member 11 is disposed on the -Z direction side of the relay member 26.

Further, the second insulator 45 is disposed on the -Z direction side of the sheet type conductive member 11.

As shown in FIG. 32, as with the first insulator 24 in Embodiment 2, the first insulator 44 includes a flat plate portion 44A of substantially rectangular shape, and a protrusion portion 44B of substantially circular cylindrical shape is formed on the flat plate portion 44A to protrude in the -Z direction. At the -Z directional end of the protrusion portion 44B, an intermediate retaining portion 44C of groove shape is formed to extend across the protrusion portion 44B in the Y direction. The intermediate retaining portion 44C is used to retain an intermediate part of the conductor portion 12A of the covered electric wire 12.

In addition, as with the first insulator 24 in Embodiment 2, the flat plate portion 44A includes the pair of through-holes 24D formed on the +Y direction side of the protrusion portion 44B and spaced apart from each other in the X direction, the insertion groove 24E formed to extend in the Y direction between the pair of through-holes 24D, the insertion groove 24F formed at the +Y directional end of the insertion groove 24E with a larger groove width than that of the insertion groove 24E, and the jutting portions 24G separately formed at the X-directional opposite lateral ends of the flat plate portion 44A and protruding in the -Z direction.

Further, the flat plate portion 44A is provided with a through-hole 44H on the -Y direction side of the protrusion portion 44B, the through-hole 44H penetrating the flat plate portion 44A in the Z direction.

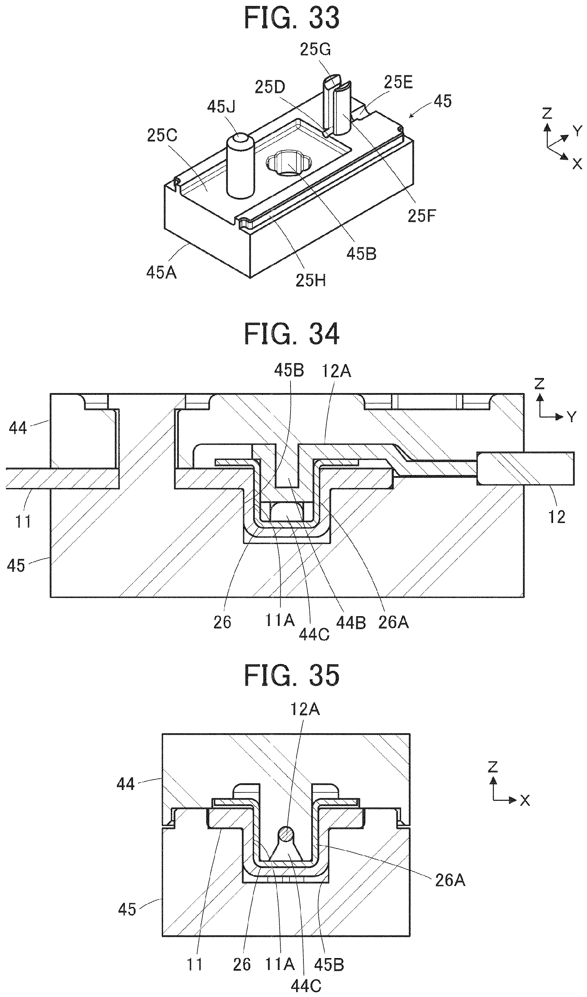

As shown in FIG. 33, as with the second insulator 25 in Embodiment 2, the second insulator 45 includes a flat plate portion 45A in which a recess portion 45B is formed.

Similarly to the second insulator 25 in Embodiment 2, the flat plate portion 45A includes the sheet type conductive member housing portion 25C of recess shape, the insertion grooves 25D and 25E, the boss 25F formed on the +Y direction side of the recess portion 45B and having the slit 25G, and the pair of step portions 25H.

Further, the flat plate portion 45A is provided with a boss 45J of circular cylindrical shape on the -Y direction side of the recess portion 45B, the boss 45J protruding in the +Z direction.

To assemble the connector according to Embodiment 4, an intermediate part of the conductor portion 12A of the covered electric wire 12 is inserted in the intermediate retaining portion 44C of the protrusion portion 44B of the first insulator 44 from the -Z direction, and in this state, the first insulator 44 is pressed toward the second insulator 45 relatively in the +Z direction with the relay member 26 and the sheet type conductive member 11 being held therebetween. Accordingly, the sheet type conductive member 11 and the conductor portion 12A of the covered electric wire 12 are held between the -Z directional surface (first retaining surface) of the first insulator 44 and the +Z directional surface (second retaining surface) of the second insulator 45.

At this time, owing to the protrusion portion 44B having a substantially circular cylindrical shape of the first insulator 44, the conductor portion 12A, the relay member 26, and the sheet type conductive member 11 are pressed in the recess portion 45B of the second insulator 45.

When the first insulator 44 is pressed against the second insulator 45, the bosses 25F and 45J of the second insulator 45 penetrate the through-holes 24D and 44H of the first insulator 44, and as shown in FIG. 29, tips of the bosses 25F and 45J protruding on the +Z direction side of the first insulator 44 are thermally deformed, whereby the first insulator 44 and the second insulator 45 are fixed to each other. Thus, the assembling operation of the connector is completed.

The protrusion portion 44B of the first insulator 44 presses the conductor portion 12A, the relay member 26, and the sheet type conductive member 11 into the recess portion 45B of the second insulator 45, whereby, as shown in FIGS. 34 and 35, the conductor portion 12A of the covered electric wire 12 deforms to conform to a surface of the protrusion portion 44B and is held between an outer surface of the protrusion portion 44B and an inner surface of the tubular portion 26A of the relay member 26. In addition, the sheet type conductive member 11 is held between an outer surface of the tubular portion 26A of the relay member 26 and an inner surface of the recess portion 45B of the second insulator 45.

Thus, the conductor portion 12A of the covered electric wire 12 is electrically connected to the flexible conductor 11A exposed on the top surface of the sheet type conductive member 11 via the relay member 26.

As with Embodiments 1 to 3, the connector according to Embodiment 4 can also reduce the size thereof while improving reliability of the electric connection between the flexible conductor 11A and the conductor portion 12A.

### Embodiment 5

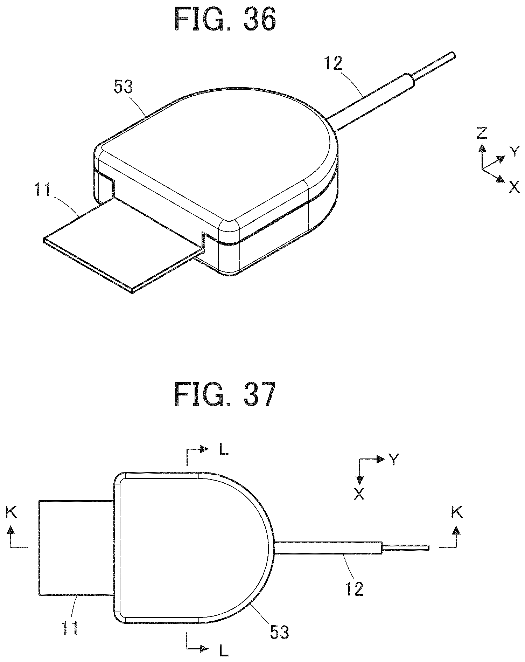

FIGS. 36 and 37 show a connector according to Embodiment 5. As with the connectors according to Embodiments 1 to 4, this connector is configured such that the covered electric wire 12 is connected to the sheet type conductive member 11, and the connector includes a housing 53 formed of an insulating resin material.

The sheet type conductive member 11 and the covered electric wire 12 are the same as those used in Embodiments 1 to 4.

In the connector according to Embodiment 5, as with the connector according to Embodiment 3, the conductor portion 12A of the covered electric wire 12 is brought in direct contact with and thus electrically connected to the flexible conductor 11A of the sheet type conductive member 11.

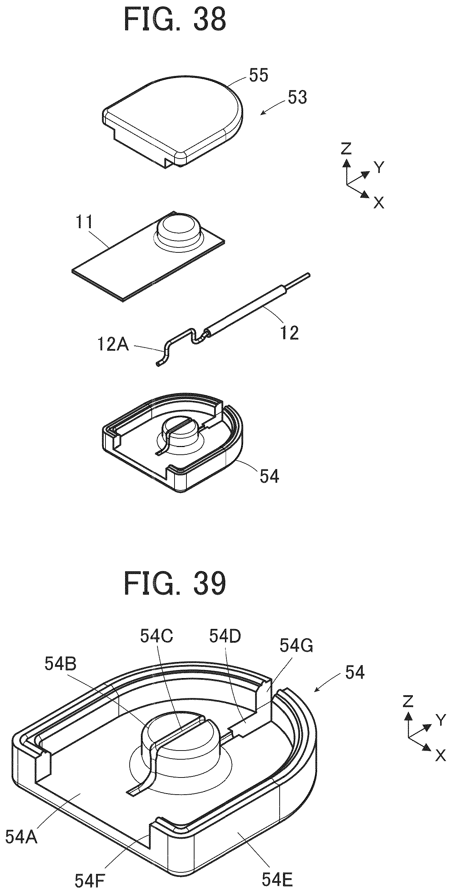

FIG. 38 shows an exploded perspective view of the connector according to Embodiment 5. The connector includes a first insulator 54 and a second insulator 55, and these first and second insulators 54 and 55 constitute the housing 53.

The conductor portion 12A of the covered electric wire 12 is disposed on the +Z direction side of the first insulator 54, the sheet type conductive member 11 is disposed on the +Z direction side of the conductor portion 12A of the covered electric wire 12, and the second insulator 55 is disposed on the +Z direction side of the sheet type conductive member 11.

As shown in FIG. 39, the first insulator 54 includes a flat plate portion 54A extending along an XY plane, and a protrusion portion 54B of substantially circular cylindrical shape protruding in the +Z direction is formed on the +Z directional surface (first retaining surface) of the flat plate portion 54A. On the upper surface of the protrusion portion 54B, an intermediate retaining portion 54C of groove shape is formed to extend across the protrusion portion 54B in the Y direction. The intermediate retaining portion 54C is used to retain an intermediate part of the conductor portion 12A of the covered electric wire 12 and extends not only on the upper surface of the protrusion portion 54B but also on lateral surfaces of the protrusion portion 54B.

On the +Z directional surface of the flat plate portion 54A, an insertion groove 54D is formed on the +Y direction side of the protrusion portion 54B, the insertion groove 54D extending in the Y direction and having a larger groove width than that of the intermediate retaining portion 54C.

Further, at a peripheral edge portion of the flat plate portion 54A, a peripheral wall 54E is formed to protrude in the +Z direction. At the -Y directional end of the flat plate portion 54A, a cutout is provided to the peripheral wall 54E to form a sheet type conductive member lead-out portion 54F, and at the +Y directional end of the flat plate portion 54A, a cutout is provided to the peripheral wall 54E to form a covered electric wire lead-out portion 54G connected to the insertion groove 54D.

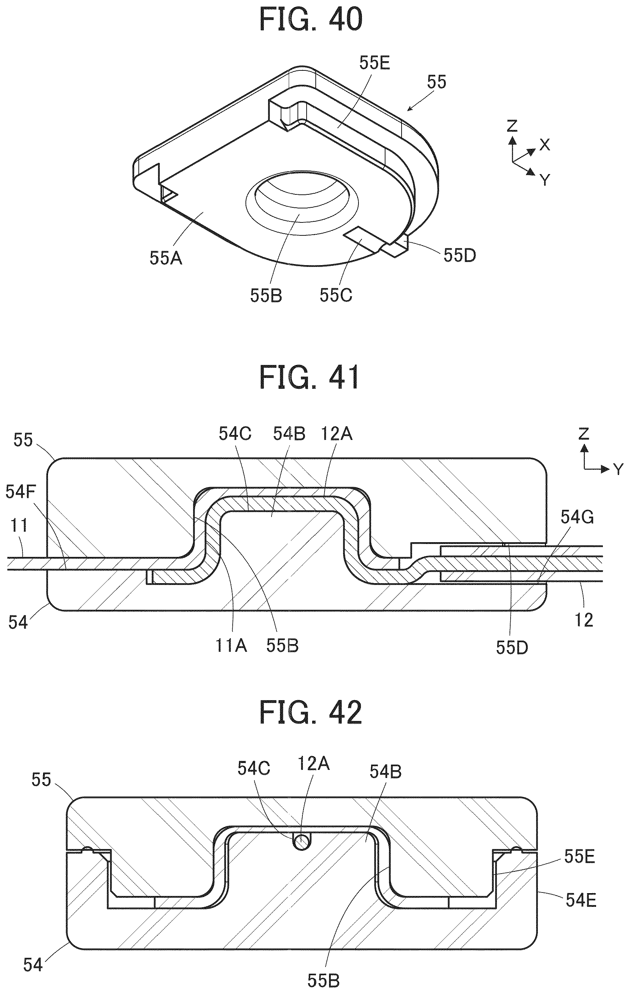

As shown in FIG. 40, the second insulator 55 includes a flat plate portion 55A extending along an XY plane, and a recess portion 55B is formed at the middle of the -Z directional surface (second retaining surface) of the flat plate portion 55A and recessed in the +Z direction.

On the -Z directional surface of the flat plate portion 55A, an insertion groove 55C is formed to extend in the Y direction from the +Y direction side of the recess portion 55B to the +Y directional end of the flat plate portion 55A, and at the +Y directional end of the flat plate portion 55A, a projection 55D is formed to be connected to the insertion groove 55C. The insertion groove 55C has the same groove width as that of the insertion groove 54D of the first insulator 54, and the projection 55D has a width corresponding to the covered electric wire lead-out portion 54G of the first insulator 54.

In addition, a step portion 55E is formed at a peripheral edge portion of the flat plate portion 55A.

To assemble the connector according to Embodiment 5, an intermediate part of the conductor portion 12A of the covered electric wire 12 is inserted in the intermediate retaining portion 54C of the protrusion portion 54B of the first insulator 54 from the +Z direction, and in this state, the first insulator 54 is pressed toward the second insulator 55 relatively in the +Z direction with the sheet type conductive member 11 being held therebetween. Accordingly, the sheet type conductive member 11 and the conductor portion 12A of the covered electric wire 12 are held between the +Z directional surface (first retaining surface) of the first insulator 54 and the -Z directional surface (second retaining surface) of the second insulator 55.

At this time, owing to the protrusion portion 54B having a substantially circular cylindrical shape of the first insulator 54, the conductor portion 12A and the sheet type conductive member 11 are pressed in the recess portion 55B of the second insulator 55.

The sheet type conductive member 11 is led out through the sheet type conductive member lead-out portion 54F of the first insulator 54, and the covered electric wire 12 is led out through the covered electric wire lead-out portion 54G of the first insulator 54.

When the second insulator 55 is pressed against the first insulator 54, the peripheral wall 54E of the first insulator 54 is fitted in the step portion 55E of the second insulator 55. Using an adhesive or the like, for instance, the peripheral wall 54E of the first insulator 54 is adhered to the step portion 55E of the second insulator 55, whereby the assembling operation of the connector is completed.

The protrusion portion 54B of the first insulator 54 presses the sheet type conductive member 11 and the conductor portion 12A into the recess portion 55B of the second insulator 55, whereby, as shown in FIGS. 41 and 42, the sheet type conductive member 11 and the conductor portion 12A deform to conform to a surface of the protrusion portion 54B and are held between an outer surface of the protrusion portion 54B and an inner surface of the recess portion 55B of the second insulator 55.

Thus, the conductor portion 12A of the covered electric wire 12 is brought into direct contact with and electrically connected to the flexible conductor 11A exposed on the top surface of the sheet type conductive member 11.

As with Embodiments 1 to 4, the connector according to Embodiment 5 can also reduce the size thereof while improving reliability of the electric connection between the flexible conductor 11A and the conductor portion 12A.

### Embodiment 6

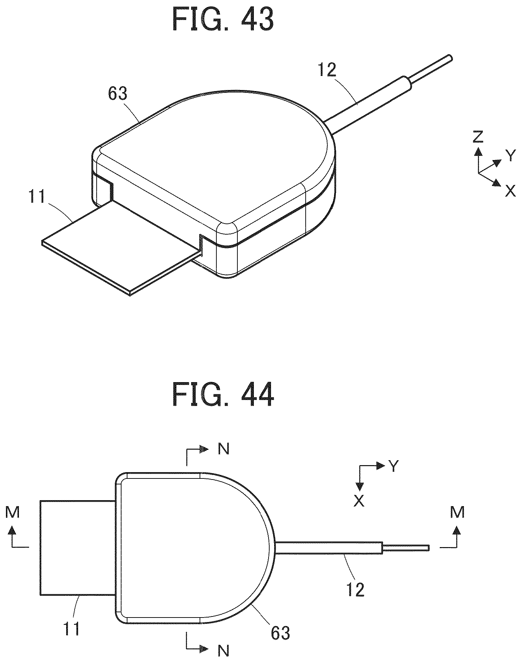

FIGS. 43 and 44 show a connector according to Embodiment 6. This connector is configured such that in the connector according to Embodiment 5, the contact force-securing member is used to secure the contact force between the conductor portion 12A of the covered electric wire 12 and the flexible conductor 11A of the sheet type conductive member 11, similarly to Embodiment 1.

The connector includes a housing 63 formed of an insulating resin material.

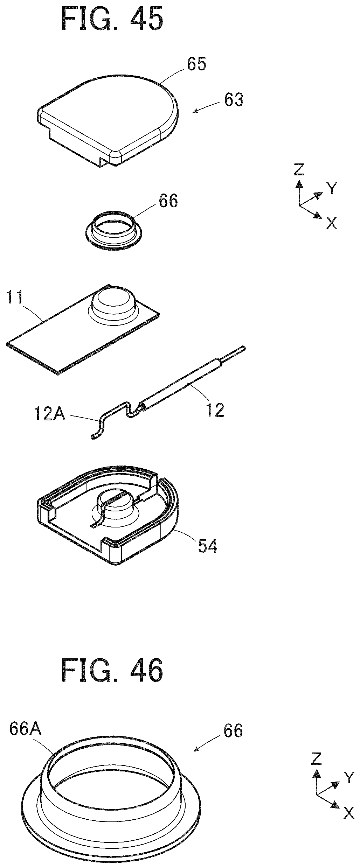

FIG. 45 shows an exploded perspective view of the connector according to Embodiment 6. The connector includes the first insulator 54 used in Embodiment 5 and a second insulator 65, and these first and second insulators 54 and 65 constitute the housing 63.

The conductor portion 12A of the covered electric wire 12 is disposed on the +Z direction side of the first insulator 54, the sheet type conductive member 11 is disposed on the +Z direction side of the conductor portion 12A of the covered electric wire 12, and a contact force-securing member 66 is disposed on the +Z direction side of the sheet type conductive member 11.

Further, the second insulator 65 is disposed on the +Z direction side of the contact force-securing member 66.

The contact force-securing member 66 is formed of a metal material and includes a tubular portion 66A of cylindrical shape as shown in FIG. 46.

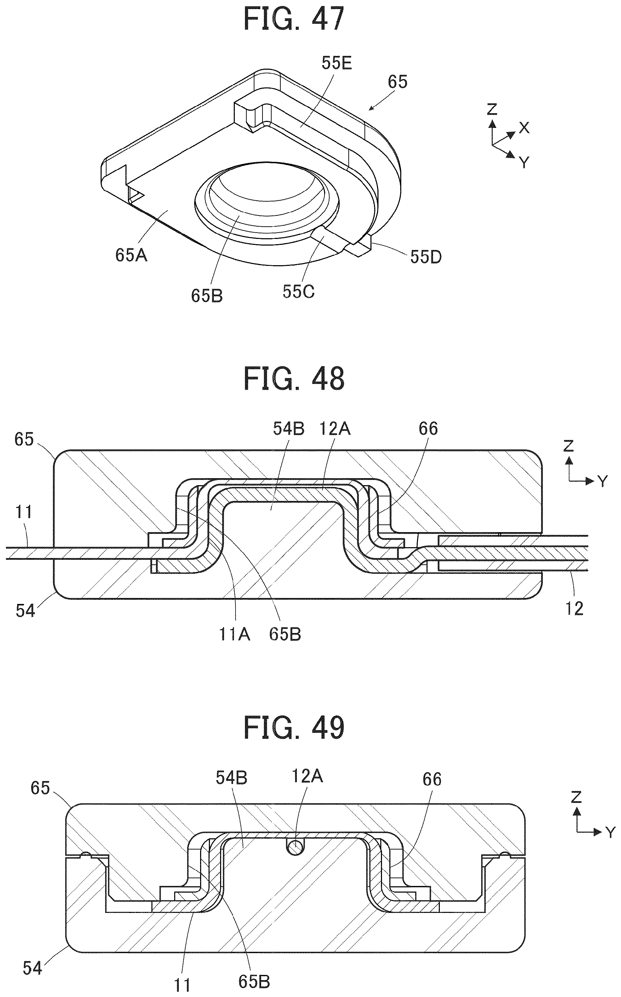

As shown in FIG. 47, as with the second insulator 55 in Embodiment 5 shown in FIG. 40, the second insulator 65 includes a flat plate portion 65A extending along an XY plane, and a recess portion 65B is formed at the middle of the -Z directional surface (second retaining surface) of the flat plate portion 65A and recessed in the +Z direction.

The recess portion 65B has a larger diameter than that of the recess portion 55B of the second insulator 55 in Embodiment 5 and is sized so that the tubular portion 66A of the contact force-securing member 66 can be housed therein.

As with the second insulator 55 in Embodiment 5, the flat plate portion 65A is provided with the insertion groove 55C, the projection 55D, and the step portion 55E.

To assemble the connector according to Embodiment 6, an intermediate part of the conductor portion 12A of the covered electric wire 12 is inserted in the intermediate retaining portion 54C of the protrusion portion 54B of the first insulator 54 from the +Z direction, while the contact force-securing member 66 is inserted in the recess portion 65B of the second insulator 65 from the -Z direction, and in this state, the first insulator 54 is pressed toward the second insulator 65 relatively in the +Z direction with the sheet type conductive member 11 being held therebetween. Accordingly, the sheet type conductive member 11 and the conductor portion 12A of the covered electric wire 12 are held between the +Z directional surface (first retaining surface) of the first insulator 54 and the -Z directional surface (second retaining surface) of the second insulator 65.

At this time, owing to the protrusion portion 54B having a substantially circular cylindrical shape of the first insulator 54, the conductor portion 12A of the covered electric wire 12 and the sheet type conductive member 11 are pressed and inserted in an inside of the contact force-securing member 66 disposed in the recess portion 65B of the second insulator 65.

When the first insulator 54 is pressed against the second insulator 65, the peripheral wall 54E of the first insulator 54 is fitted in the step portion 55E of the second insulator 65. Using an adhesive or the like, for instance, the peripheral wall 54E of the first insulator 54 is adhered to the step portion 55E of the second insulator 65, whereby the assembling operation of the connector is completed.

The protrusion portion 54B of the first insulator 54 is inserted in the inside of the contact force-securing member 66 while pressing the sheet type conductive member 11 and the conductor portion 12A of the covered electric wire 12 therein, whereby, as shown in FIGS. 48 and 49, the sheet type conductive member 11 and the conductor portion 12A deform to conform to a surface of the protrusion portion 54B and are held between an outer surface of the protrusion portion 54B and an inner surface of the contact force-securing member 66.

With the contact force-securing member 66 being disposed on an outside of the conductor portion 12A of the covered electric wire 12 and the sheet type conductive member 11 in the recess portion 65 as above, the conductor portion 12A of the covered electric wire 12 is brought into contact with the flexible conductor 11A exposed on the top surface of the sheet type conductive member 11 at a predetermined contact force and is electrically connected to the flexible conductor 11A.

As with Embodiments 1 to 5, the connector according to Embodiment 6 can also reduce the size thereof while improving reliability of the electric connection between the flexible conductor 11A and the conductor portion 12A.

While the covered electric wire 12 is used as an electric wire to be connected to the sheet type conductive member 11 in Embodiments 1 to 6 above, an electric wire formed of only the conductor portion 12A whose outer periphery is not covered with the insulating cover portion 12B made of an insulating material may also be connected to the sheet type conductive member 11.

In addition, in Embodiments 1 to 6 above, one protrusion portion 14B, 24B, 44B, 54B formed on the first insulator 14, 24, 44, 54 is housed in one recess portion 15C, 25B, 35B, 45B, 55B, 65B formed in the second insulator 15, 25, 35, 45, 55, 65 to thereby connect the conductor portion 12A of one covered electric wire 12 to the flexible conductor 11A of one sheet type conductive member 11, but the connector can be configured such that, using the first insulator having a plurality of protrusion portions and the second insulator having a plurality of recess portions, conductor portions of a plurality of electric wires are connected to flexible conductors of a plurality of sheet type conductive members in a similar manner.

## Claims

1. A connector for connecting a conductor portion (12A) of an electric wire (12) to a flexible conductor (11A) exposed on at least one surface of a sheet type conductive member (11), the connector comprising:
a first insulator (14, 24, 44, 54) including a first retaining surface and a protrusion portion (14B, 24B, 44B, 54B) formed on and protruding from the first retaining surface; and
a second insulator (15, 25, 35, 45, 55, 65) including a second retaining surface opposed to the first retaining surface and a recess portion (15C, 25B, 35B, 45B, 55B, 65B) formed in the second retaining surface and corresponding to the protrusion portion,
wherein the first insulator and the second insulator are connected to each other with the sheet type conductive member and the electric wire being held between the first retaining surface and the second retaining surface, and
at least part of the protrusion portion is housed in the recess portion, whereby the conductor portion of the electric wire is electrically connected to the flexible conductor of the sheet type conductive member in the recess portion,
wherein one of the following options applies:
- the protrusion portion (14B, 24B) has a prismatic shape, and the recess portion (15C, 25B, 35B) has a cylindrical inner surface,
- the protrusion portion (44B, 54B) has a circular cylindrical shape, and the recess portion (45B, 55B, 65B) has a cylindrical inner surface.

2. The connector according to claim 1,
wherein the protrusion portion (44B, 54B) has an intermediate retaining portion (44C, 54C) of groove shape for retaining an intermediate part of the conductor portion of the electric wire.

3. The connector according to any one of claims 1 and 2, further comprising a relay member (26) having conductivity and disposed in the recess portion (25B, 45B), and
wherein the conductor portion of the electric wire is electrically connected to the flexible conductor via the relay member.

4. The connector according to claim 3,
wherein the relay member (26) includes a tubular portion (26A) of cylindrical shape,
the flexible conductor (11A) is held between an outer surface of the protrusion portion (24B) and an inner surface of the tubular portion (26A), and
the conductor portion (12A) of the electric wire is held between an outer surface of the tubular portion (26A) and an inner surface of the recess portion (25B).

5. The connector according to claim 3,
wherein the relay member (26) includes a tubular portion (26A) of cylindrical shape,
the conductor portion (12A) of the electric wire is held between an outer surface of the protrusion portion (44B) and an inner surface of the tubular portion (26A), and
the flexible conductor (11A) is held between an outer surface of the tubular portion (26A) and an inner surface of the recess portion (45B).

6. The connector according to any one of claims 1 and 2, wherein the conductor portion (12A) of the electric wire is brought into direct contact with and electrically connected to the flexible conductor (11A).

7. The connector according to claim 6, further comprising a contact force-securing member (16, 66) for securing a contact force between the conductor portion and the flexible conductor, the contact force-securing member being disposed on an outside of the conductor portion (12A) and the flexible conductor (11A) that are in contact with each other in the recess portion (15C, 65B).

8. The connector according to claim 7, wherein the contact force-securing member (16, 66) is made of a metal material and has a cylindrical shape.

9. The connector according to any one of claims 1 and 2,
wherein the sheet type conductive member (11) extends in a predetermined direction (-Y) from the first insulator and the second insulator along the first retaining surface and the second retaining surface, and
the electric wire (12) extends in an opposite direction (+Y) of the predetermined direction from the first insulator and the second insulator along the first retaining surface and the second retaining surface.

10. The connector according to any one of claims 1 and 2, wherein one of the first insulator (14) and the second insulator (15) includes a tip retaining portion (15G, 25G) for retaining a tip of the conductor portion of the electric wire.

11. The connector according to claim 10, wherein the tip retaining portion includes a slit (15G, 25G) in which the tip of the conductor portion is inserted.

12. The connector according to claim 10,
wherein the electric wire (12) includes an insulating cover portion (12B) covering an outer periphery of the conductor portion, and
the first insulator and the second insulator have an insulating cover retaining portion (14D, 15E) for retaining the insulating cover portion of the electric wire.

13. The connector according to any one of claims 1 and 2,
wherein one of the first insulator and the second insulator includes a boss (15H, 25F, 45J),
another one of the first insulator and the second insulator includes a through-hole (14F, 24C, 24D, 44H) which the boss penetrates, and
a head of the boss having penetrated the through-hole is thermally deformed.

14. The connector according to claim 13, wherein the boss includes a slit (15G, 25G) in which the conductor portion of the electric wire is inserted.

## Patentansprüche

1. Verbinder zum Verbinden eines Leiterabschnitts (12A) eines elektrischen Drahts (12) mit einem flexiblen Leiter (11A), der an mindestens einer Oberfläche eines plattenartigen leitfähigen Elements (11) freiliegt, wobei der Verbinder umfasst:
einen ersten Isolator (14, 24, 44, 54) mit einer ersten Haltefläche und einem Vorsprungsabschnitt (14B, 24B, 44B, 54B), der an der ersten Haltefläche ausgebildet ist und von dieser vorsteht; und
einen zweiten Isolator (15, 25, 35, 45, 55, 65) mit einer zweiten Haltefläche gegenüber der ersten Haltefläche und einem Aussparungsabschnitt (15C, 25B, 35B, 45B, 55B, 65B), der in der zweiten Haltefläche ausgebildet ist und dem Vorsprungsabschnitt entspricht,
wobei der erste Isolator und der zweite Isolator miteinander verbunden sind, wobei das plattenartige leitfähige Element und der elektrische Draht zwischen der ersten Haltefläche und der zweiten Haltefläche gehalten sind, und
mindestens ein Teil des Vorsprungsabschnitts in dem Aussparungsabschnitt aufgenommen ist, wodurch der Leiterabschnitt des elektrischen Drahts mit dem flexiblen Leiter des plattenartigen leitfähigen Elements in dem Aussparungsabschnitt elektrisch verbunden ist,
wobei eine der folgenden Optionen zutrifft:
- der Vorsprungsabschnitt (14B, 24B) hat eine prismatische Form, und der Aussparungsabschnitt (15C, 25B, 35B) hat eine zylindrische Innenfläche,
- der Vorsprungsabschnitt (44B, 54B) hat eine kreiszylindrische Form, und der Aussparungsabschnitt (45B, 55B, 65B) hat eine zylindrische Innenfläche.

2. Verbinder nach Anspruch 1,
wobei der Vorsprungsabschnitt (44B, 54B) einen Zwischenhalteabschnitt (44C, 54C) mit einer Nutform zum Halten eines Zwischenteils des Leiterabschnitts des elektrischen Drahts hat.

3. Verbinder nach einem der Ansprüche 1 und 2, ferner umfassend ein Relaiselement (26), das Leitfähigkeit hat und in dem Aussparungsabschnitt (25B, 45B) angeordnet ist, und
wobei der Leiterabschnitt des elektrischen Drahts über das Relaiselement mit dem flexiblen Leiter elektrisch verbunden ist.

4. Verbinder nach Anspruch 3,
wobei das Relaiselement (26) einen röhrenförmigen Abschnitt (26A) mit einer zylindrischen Form aufweist,
der flexible Leiter (11A) zwischen einer Außenfläche des Vorsprungsabschnitts (24B) und einer Innenfläche des röhrenförmigen Abschnitts (26A) gehalten ist, und
der Leiterabschnitt (12A) des elektrischen Drahts zwischen einer Außenfläche des röhrenförmigen Abschnitts (26A) und einer Innenfläche des Aussparungsabschnitts (25B) gehalten ist.

5. Verbinder nach Anspruch 3,
wobei das Relaiselement (26) einen röhrenförmigen Abschnitt (26A) mit einer zylindrischen Form aufweist,
der Leiterabschnitt (12A) des elektrischen Drahts zwischen einer Außenfläche des Vorsprungsabschnitts (44B) und einer Innenfläche des röhrenförmigen Abschnitts (26A) gehalten ist, und
der flexible Leiter (11A) zwischen einer Außenfläche des röhrenförmigen Abschnitts (26A) und einer Innenfläche des Aussparungsabschnitts (45B) gehalten ist.

6. Verbinder nach einem der Ansprüche 1 und 2, wobei der Leiterabschnitt (12A) des elektrischen Drahts in direkten Kontakt mit dem flexiblen Leiter (11A) gebracht und mit diesem elektrisch verbunden ist.

7. Verbinder nach Anspruch 6, ferner umfassend ein Kontaktkraftsicherungselement (16, 66) zum Sichern einer Kontaktkraft zwischen dem Leiterabschnitt und dem flexiblen Leiter, wobei das Kontaktkraftsicherungselement an einer Außenseite des Leiterabschnitts (12A) und des flexiblen Leiters (11A) angeordnet ist, die in dem Aussparungsabschnitt (15C, 65B) miteinander in Kontakt stehen.

8. Verbinder nach Anspruch 7, wobei das Kontaktkraftsicherungselement (16, 66) aus einem Metallmaterial hergestellt ist und eine zylindrische Form hat.

9. Verbinder nach einem der Ansprüche 1 und 2,
wobei sich das plattenartige leitfähige Element (11) in einer vorbestimmten Richtung (-Y) von dem ersten Isolator und dem zweiten Isolator entlang der ersten Haltefläche und der zweiten Haltefläche erstreckt, und
sich der elektrische Draht (12) in einer entgegengesetzten Richtung (+Y) zu der vorbestimmten Richtung von dem ersten Isolator und dem zweiten Isolator entlang der ersten Haltefläche und der zweiten Haltefläche erstreckt.

10. Verbinder nach einem der Ansprüche 1 und 2, wobei einer von dem ersten Isolator (14) und dem zweiten Isolator (15) einen Spitzenhalteabschnitt (15G, 25G) zum Halten einer Spitze des Leiterabschnitts des elektrischen Drahts aufweist.

11. Verbinder nach Anspruch 10, wobei der Spitzenhalteabschnitt einen Schlitz (15G, 25G) aufweist, in den die Spitze des Leiterabschnitts eingeführt ist.

12. Verbinder nach Anspruch 10,
wobei der elektrische Draht (12) einen Isolierende-Abdeckung-Abschnitt (12B) aufweist, der einen Außenumfang des Leiterabschnitts abdeckt, und
der erste Isolator und der zweite Isolator einen Isolierende-Abdeckung-HalteAbschnitt (14D, 15E) zum Halten des Isolierende-Abdeckung-Abschnitts des elektrischen Drahts haben.

13. Verbinder nach einem der Ansprüche 1 und 2,
wobei einer von dem ersten Isolator und dem zweiten Isolator einen Vorsprung (15H, 25F, 45J) aufweist,
ein anderer von dem ersten Isolator und dem zweiten Isolator ein Durchgangsloch (14F, 24C, 24D, 44H) aufweist, das der Vorsprung durchdringt, und
ein Kopf des Vorsprungs, der das Durchgangsloch penetriert hat, thermisch verformt wird.

14. Verbinder nach Anspruch 13, wobei der Vorsprung einen Schlitz (15G, 25G) aufweist, in den der Leiterabschnitt des elektrischen Drahts eingeführt ist.

## Revendications

1. Connecteur pour connecter une partie conductrice (12A) d'un fil électrique (12) à un conducteur flexible (11A) exposé sur au moins une surface d'un élément conducteur de type feuille (11), le connecteur comprenant :
un premier isolateur (14, 24, 44, 54) comprenant une première surface de retenue et une partie en saillie (14B, 24B, 44B, 54B) formée sur et faisant saillie de la première surface de retenue ; et
un second isolateur (15, 25, 35, 45, 55, 65) comprenant une seconde surface de retenue opposée à la première surface de retenue et une partie évidée (15C, 25B, 35B, 45B, 55B, 65B) formée dans la seconde surface de retenue et correspondant à la partie en saillie,
dans lequel le premier isolateur et le second isolateur sont connectés l'un à l'autre avec l'élément conducteur de type feuille et le fil électrique maintenu entre la première surface de retenue et la seconde surface de retenue, et
au moins une partie de la partie en saillie est logée dans la partie évidée, moyennant quoi la partie conductrice du fil électrique est connectée électriquement au conducteur flexible de l'élément conducteur de type feuille dans la partie évidée,
dans lequel l'une des options suivantes s'applique :
- la partie en saillie (14B, 24B) a une forme prismatique, et la partie évidée (15C, 25B, 35B) a une surface interne cylindrique,
- la partie en saillie (44B, 54B) a une forme cylindrique circulaire, et la partie évidée (45B, 55B, 65B) a une surface interne cylindrique.

2. Connecteur selon la revendication 1,
dans lequel la partie en saillie (44B, 54B) a une partie de retenue intermédiaire (44C, 54C) en forme de rainure pour retenir une partie intermédiaire de la partie conductrice du fil électrique.

3. Connecteur selon l'une quelconque des revendications 1 et 2, comprenant en outre un élément de relais (26) ayant une conductivité et disposé dans la partie évidée (25B, 45B), et
dans lequel la partie conductrice du fil électrique est connectée électriquement au conducteur flexible via l'élément de relais.

4. Connecteur selon la revendication 3,
dans lequel l'élément de relais (26) comprend une partie tubulaire (26A) de forme cylindrique,
le conducteur flexible (1 1A) est maintenu entre une surface externe de la partie en saillie (24B) et une surface interne de la partie tubulaire (26A), et
la partie conductrice (12A) du fil électrique est maintenue entre une surface externe de la partie tubulaire (26A) et une surface interne de la partie évidée (25B).

5. Connecteur selon la revendication 3,
dans lequel l'élément de relais (26) comprend une partie tubulaire (26A) de forme cylindrique,
la partie conductrice (12A) du fil électrique est maintenue entre une surface externe de la partie en saillie (44B) et une surface interne de la partie tubulaire (26A), et
le conducteur flexible (11A) est maintenu entre une surface externe de la partie tubulaire (26A) et une surface interne de la partie évidée (45B).

6. Connecteur selon l'une quelconque des revendications 1 et 2, dans lequel la partie conductrice (12A) du fil électrique est amenée en contact direct avec et connectée électriquement au conducteur flexible (11A).

7. Connecteur selon la revendication 6, comprenant en outre un élément de sécurisation de force de contact (16, 66) pour assurer une force de contact entre la partie conductrice et le conducteur flexible, l'élément de sécurisation de force de contact étant disposé sur un extérieur de la partie conductrice (12A) et du conducteur flexible (11A) qui sont en contact l'un avec l'autre dans la partie évidée (15C, 65B).

8. Connecteur selon la revendication 7, dans lequel l'élément de fixation de force de contact (16, 66) est constitué d'un matériau métallique et a une forme cylindrique.

9. Connecteur selon l'une quelconque des revendications 1 et 2,
dans lequel l'élément conducteur de type feuille (11) s'étend dans une direction prédéterminée (-Y) à partir du premier isolateur et du second isolateur le long de la première surface de retenue et de la seconde surface de retenue, et
le fil électrique (12) s'étend dans une direction opposée (+Y) à la direction prédéterminée à partir du premier isolateur et du second isolateur le long de la première surface de retenue et de la seconde surface de retenue.

10. Connecteur selon l'une quelconque des revendications 1 et 2, dans lequel l'un du premier isolateur (14) et du second isolateur (15) comprend une partie de retenue de pointe (15G, 25G) pour retenir une pointe de la partie conductrice du fil électrique.

11. Connecteur selon la revendication 10, dans lequel la partie de retenue de pointe comprend une fente (15G, 25G) dans laquelle la pointe de la partie conductrice est insérée.

12. Connecteur selon la revendication 10,
dans lequel le fil électrique (12) comprend une partie de couverture isolante (12B) couvrant une périphérie externe de la partie conductrice, et
le premier isolateur et le second isolateur ont une partie de retenue de couverture isolante (14D, 15E) pour retenir la partie de couverture isolante du fil électrique.

13. Connecteur selon l'une quelconque des revendications 1 et 2,
dans lequel l'un du premier isolateur et du second isolateur comprend un bossage (15H, 25F, 45J),
un autre du premier isolateur et du second isolateur comprend un trou traversant (14F, 24C, 24D, 44H) dans lequel pénètre le bossage, et
une tête du bossage ayant pénétré dans le trou traversant est déformée thermiquement.

14. Connecteur selon la revendication 13, dans lequel le bossage comprend une fente (15G, 25G) dans laquelle la partie conductrice du fil électrique est insérée.
